# EUROPEAN PATENT APPLICATION

(11) **EP 4 645 319 A1**
(43) Date of publication of application: **05.11.2025**
(21) Application number: 23912308.6
(22) Date of filing: 28.12.2023
(51) Int. Cl.: G16B 30/00

(54) **SEQUENCE EXTRACTION SYSTEM, SEQUENCE EXTRACTION METHOD, AND SEQUENCE EXTRACTION PROGRAM**

(30) Priority: 28.12.2022 JP 2022212061
(71) Applicant: The University of Tokyo, Bunkyo-ku, Tokyo 113-8654 (JP)
(72) Inventor: KATO, Hideaki, Tokyo 113-8654 (JP); KOJIMA, Asato, Tokyo 113-8654 (JP); MATSUI, Toshiki, Tokyo 113-8654 (JP)
(74) Representative: D Young & Co LLP
(86) International application number: PCT/JP2023/047216
(87) International publication number: WO 2024/143531

(57) **Abstract**

A sequence extraction system includes a candidate sequence generation unit that generates a plurality of candidate fusion sequences, which are a plurality of candidates for a fusion sequence in which at least one inserted amino acid sequence corresponding to insertion polypeptide is inserted into an entirety or a part of a target amino acid sequence corresponding to a target protein, based on each of a plurality of candidate insertion patterns, which are a plurality of candidates for an insertion pattern for inserting at least one of the inserted amino acid sequence into the entirety or the part of the target amino acid sequence, a structure prediction result acquisition unit that acquires structure prediction result information indicating a result of prediction of a structure of each of a plurality of candidate fusion proteins corresponding to the plurality of candidate fusion sequences respectively, based on each of the plurality of candidate fusion sequences, an evaluation unit that evaluates an insertion site into which the inserted amino acid sequence is inserted, based on the result of the prediction, and a sequence extraction unit that extracts at least one candidate fusion sequence from the plurality of candidate fusion sequences, based on the result of the evaluation.

## Description

### Technical Field

The present invention relates to a sequence extraction system, a sequence extraction method, and a sequence extraction program.

### Background Art

In the related art, there is known a method of analyzing a three-dimensional structure of proteins by combining biochemical experiments with computer-based analysis.

For example, in a protein structure analysis method disclosed in Patent Literature 1, a target protein whose three-dimensional structure is to be predicted is fragmented to measure a fragmentation spectrum, and assignment information of fragmentation ions is determined with respect to an amino acid sequence of the target protein on the basis of the fragmentation spectrum. In the method, a site that is in contact with a solvent in the amino acid sequence of the target protein is specified in accordance with the fragmentation spectrum and the assignment information of fragmentation ions, and solvent accessible residue information in the amino acid sequence of the target protein is determined in accordance with the site. Furthermore, in the method, a three-dimensional structure of the target protein is predicted, reference vibration calculation is performed for the target protein, and the predicted three-dimensional structure prediction data, the solvent accessible residue information, and the calculation results of the reference vibration calculation are output in association with each other.

### Citation List

### Patent Literature

Patent Literature 1: Patent Publication JP-A-2008-76406

### Summary of Invention

### Technical Problem

In this regard, in three-dimensional structure analysis for a protein, it is necessary to select a protein suitable for three-dimensional structure analysis. A protein suitable for three-dimensional structure analysis is selected through, for example, selection of a biological species, editing of an amino acid sequence of a protein (for example, mutation, insertion or deletion of the amino acid sequence, and the like), examination of complexes with compounds (for example, agonists or antagonists) or different proteins, and examination of expression and purification methods. That is, in the three-dimensional structure analysis for a protein, a protein suitable for three-dimensional structure analysis is selected from among a plurality of candidate proteins.

However, in the protein structure analysis method disclosed in Patent Literature 1, it is necessary to perform the method for each of the target proteins whose three-dimensional structures are to be predicted. For this reason, in a case where there are a plurality of candidate proteins, this places a heavy burden on a user.

Consequently, an object of the present invention is to provide a sequence extraction system, a sequence extraction method, and a sequence extraction program which are capable of easily extracting an amino acid sequence of a protein suitable for three-dimensional structure analysis.

### Solution to Problem

A sequence extraction system according to one embodiment of the present invention includes a candidate sequence generation unit that generates a plurality of candidate fusion sequences, which are a plurality of candidates for a fusion sequence in which at least one inserted amino acid sequence corresponding to insertion polypeptide is inserted into an entirety or a part of a target amino acid sequence corresponding to a target protein, based on each of a plurality of candidate insertion patterns, which are a plurality of candidates for an insertion pattern for inserting at least one of the inserted amino acid sequence into the entirety or the part of the target amino acid sequence, a structure prediction result acquisition unit that acquires structure prediction result information indicating a result of prediction of a structure of each of a plurality of candidate fusion proteins corresponding to the plurality of candidate fusion sequences, based on each of the plurality of candidate fusion sequences, an evaluation unit that evaluates an insertion site into which the inserted amino acid sequence is inserted, based on the result of the prediction, and a sequence extraction unit that extracts at least one candidate fusion sequence from the plurality of candidate fusion sequences based on the result of the evaluation.

A sequence extraction method according to one embodiment of the present invention is executed by a computer, the method comprising: generating a plurality of candidate fusion sequences, which are a plurality of candidates for a fusion sequence in which at least one inserted amino acid sequence corresponding to insertion polypeptide is inserted into an entirety or a part of a target amino acid sequence corresponding to a target protein, based on each of a plurality of candidate insertion patterns, which are a plurality of candidates for an insertion pattern for inserting at least one of the inserted amino acid sequence into the entirety or the part of the target amino acid sequence; acquiring structure prediction result information indicating a result of prediction of a structure of each of a plurality of candidate fusion proteins corresponding to the plurality of candidate fusion sequences respectively, based on each of the plurality of candidate fusion sequences; evaluating an insertion site into which the inserted amino acid sequence is inserted, based on the result of the prediction; and extracting at least one candidate fusion sequence from the plurality of candidate fusion sequences, based on the result of the evaluation.

A sequence extraction program according to one embodiment of the present invention causes a computer to implement: a candidate sequence generation unit that generates a plurality of candidate fusion sequences, which are a plurality of candidates for a fusion sequence in which at least one inserted amino acid sequence corresponding to insertion polypeptide is inserted into an entirety or a part of a target amino acid sequence corresponding to a target protein, based on each of a plurality of candidate insertion patterns, which are a plurality of candidates for an insertion pattern for inserting at least one of the inserted amino acid sequence into the entirety or the part of the target amino acid sequence; a structure prediction result acquisition unit that acquires structure prediction result information indicating a result of prediction of a structure of each of a plurality of candidate fusion proteins corresponding to the plurality of candidate fusion sequences respectively, based on each of the plurality of candidate fusion sequences; an evaluation unit that evaluates an insertion site into which the inserted amino acid sequence is inserted, based on the result of the prediction; and a sequence extraction unit that extracts at least one candidate fusion sequence from the plurality of candidate fusion sequences, based on the result of the evaluation.

Note that, in the present invention, a "unit" does not simply mean a physical means, but also includes cases where the function of the "unit" is implemented by software. In addition, the function of one "unit" or device may be implemented by two or more physical means, devices, or software, and the functions of two or more "units" or devices may be implemented by one physical means, device, or software.

### Advantageous Effect of Invention

According to the present invention, it is possible to provide a sequence extraction system, a sequence extraction method, and a sequence extraction program which are capable of easily extracting an amino acid sequence of a protein suitable for three-dimensional structure analysis.

### Brief Description of Drawings

[Figure 1] Figure 1 is a diagram showing an overview of processing in a sequence extraction system 100 according to an embodiment of the present invention.
[Figure 2] Figure 2 is a diagram showing the configuration of the sequence extraction system 100 according to an embodiment of the present invention.
[Figure 3A] Figure 3A is a diagram showing an example of target amino acid sequence information stored in a storage unit 110.
[Figure 3B] Figure 3B is a diagram showing an example of inserted amino acid sequence information stored in the storage unit 110.
[Figure 4] Figure 4 is a diagram showing an example of an insertion pattern.
[Figure 5] Figure 5 is a diagram showing an example of candidate fusion sequence information stored in the storage unit 110.
[Figure 6] Figure 6 is a diagram showing an example of structure prediction result information stored in the storage unit 110.
[Figure 7] Figure 7 is a diagram showing an example of evaluation result information stored in the storage unit 110.
[Figure 8] Figure 8 is a flowchart showing an example of processing in the sequence extraction system 100.
[Figure 9] Figure 9 is a diagram showing an example of a first angle.
[Figure 10] Figure 10 is a diagram showing an example of a hardware configuration of a computer 1000.

### Description of Embodiments

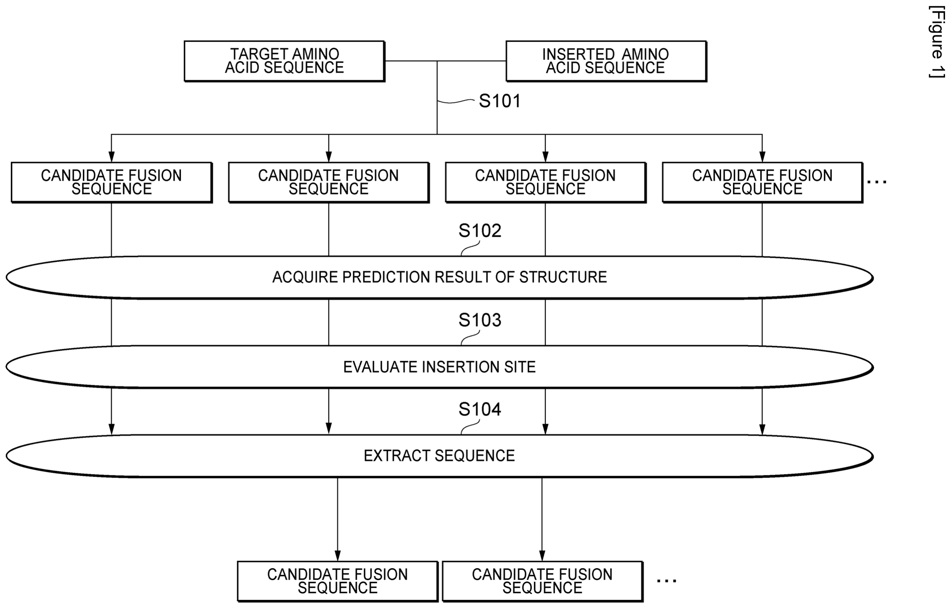

A preferred embodiment of the present invention will be described with reference to the accompanying drawings. Figure 1 is a diagram showing an overview of processing in a sequence extraction system 100 according to an embodiment of the present invention.

The sequence extraction system 100 is an information processing system implemented by a sequence extraction program, and is an information processing system that evaluates a plurality of candidate fusion sequences in which an inserted amino acid sequence of an insertion polypeptide is inserted into an amino acid sequence of a target protein, and extracts at least one candidate fusion sequence from the plurality of candidate fusion sequences.

An amino acid sequence of a wild-type target protein may not be suitable for three-dimensional structure analysis of a target protein. This may occur, for example, when the molecular weight of the target protein is small or when the thermal stability of the target protein is low.

In such cases, three-dimensional structure analysis of a target protein may be performed using a fusion sequence in which an inserted amino acid sequence of an insertion polypeptide is inserted into an amino acid sequence of the target protein. However, a plurality of (or infinite) insertion patterns for inserting an insertion polypeptide are assumed. The insertion patterns may directly affect a success or failure in three-dimensional structure analysis of a target protein.

Consequently, the sequence extraction system 100 evaluates a plurality of candidate fusion sequences corresponding to a plurality of insertion patterns and extracts at least one candidate fusion sequence suitable for three-dimensional structure analysis of a target protein.

First, the sequence extraction system 100 acquires a target amino acid sequence and an inserted amino acid sequence to generate a plurality of candidate fusion sequences on the basis of each of the plurality of candidate insertion patterns, which are a plurality of candidates for an insertion pattern to be inserted (S101).

Subsequently, the sequence extraction system 100 acquires prediction results of the respective structures of the plurality of candidate fusion proteins corresponding to the plurality of candidate fusion sequences (S102). The sequence extraction system 100 evaluates an insertion site on the basis of the prediction results (S103).

Then, the sequence extraction system 100 extracts at least one candidate fusion sequence from the plurality of candidate fusion sequences on the basis of the evaluation results (S104).

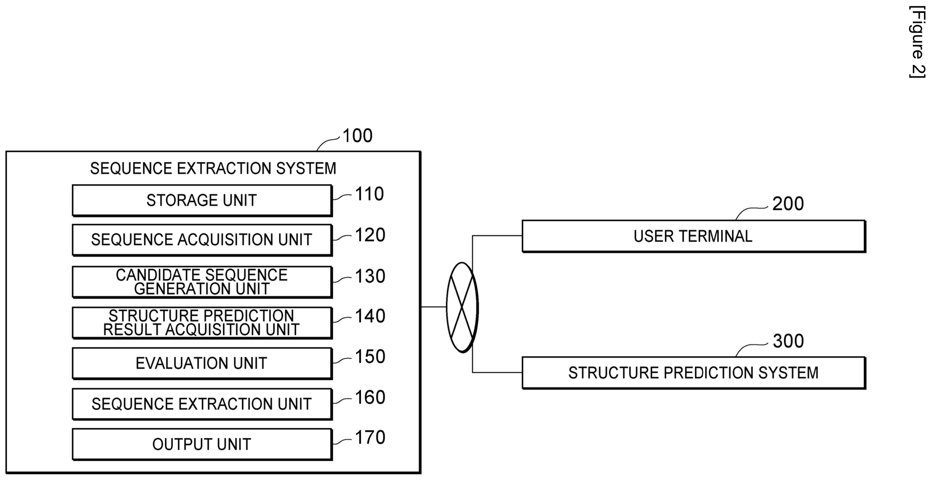

Figure 2 is a diagram showing the configuration of the sequence extraction system 100 according to an embodiment of the present invention. The sequence extraction system 100 is communicably connected to a user terminal 200 and a structure prediction system 300 via a network such as the Internet. Details of the sequence extraction system 100 will be described later.

The user terminal 200 is a computer used by a user, such as a smartphone, a tablet terminal, or a personal computer.

The user accesses the sequence extraction system 100 by using the user terminal 200, and provides, for example, a target amino acid sequence and an inserted amino acid sequence. The user also accesses the sequence extraction system 100 by using the user terminal 200, and acquires, for example, at least one candidate fusion sequence extracted in the sequence extraction system 100.

The structure prediction system 300 acquires candidate fusion sequences from the sequence extraction system 100 to predict the structures of a plurality of candidate fusion proteins corresponding to the candidate fusion sequences, and provides the prediction results to the sequence extraction system 100.

Note that, although one user terminal 200 and one structure prediction system 300 are shown in Figure 2, there may be a plurality of user terminals 200 and a plurality of structure prediction systems 300.

Subsequently, the sequence extraction system 100 will be described in detail. The sequence extraction system 100 includes a storage unit 110, a sequence acquisition unit 120, a candidate sequence generation unit 130, a structure prediction result acquisition unit 140, an evaluation unit 150, a sequence extraction unit 160, and an output unit 170. The units shown in Figure 2 can be implemented, for example, by using a storage area or by a processor executing a program stored in the storage area.

The storage unit 110 stores information processed in the sequence extraction system 100. The storage unit 110 can store, for example, target amino acid sequence information, inserted amino acid sequence information, candidate fusion sequence information, structure prediction result information, and evaluation result information, which will be described later.

The sequence acquisition unit 120 acquires target amino acid sequence information on a target amino acid sequence of a target protein, and inserted amino acid sequence information on an inserted amino acid sequence of an insertion polypeptide, and stores them in the storage unit 110.

The target protein is a protein that is a target for three-dimensional structure analysis by the user (for example, analysis using X-ray crystal structure analysis or a cryo-electron microscope). The target amino acid sequence is an amino acid sequence of the target protein.

The target protein may be, for example, a membrane protein located on a cell membrane.

The target protein may also be, for example, a G protein-coupled receptor (GPCR).

There are G protein-coupled receptors in an active state and in an inactive state. When an agonist is bonded to a GPCR, the GPCR is set to be in an active state and is bonded to a trimeric G protein. When the GPCR is not bonded to an agonist or is bonded to an antagonist, the GPCR is set to be in an inactive state and is not bonded to a trimeric G protein.

In recent years, it has become known that a GPCR plays a wide variety of important roles in a living body, and three-dimensional structure analysis of a GPCR using a cryo-electron microscope has attracted attention. While the three-dimensional structure analysis using the cryo-electron microscopy is suitable for three-dimensional structure analysis of proteins having a certain size or larger, it may not be suitable for three-dimensional structure analysis of proteins having a certain size or smaller or proteins whose protruding parts from surfactant micelles have a certain size or smaller. That is, GPCRs alone may not be suitable for three-dimensional structure analysis using a cryo-electron microscope.

For this reason, a large number of three-dimensional structure analyses using a cryo-electron microscope have been performed on agonist-coupled GPCRs, in which an agonist is bonded to a GPCR and the GPCR is complexed with a trimeric G protein. On the other hand, an inactive GPCR is not bonded to a trimeric G protein, making it difficult to clarify the structure of the inactive GPCR (particularly, the mode of bonding to an antagonist).

Due to the above problems, in three-dimensional structure of an inactive GPCR, three-dimensional structure analysis using a cryo-electron microscope may be performed on a fusion protein in which an insertion polypeptide (for example, a protein) is inserted into a specific position in the GPCR.

However, countless possible insertion patterns for inserting an insertion polypeptide into a specific position in the GPCR are assumed. In addition, for proteins that easily denature, even a slight difference in an insertion pattern greatly affects the thermal stability of a fusion protein, and ultimately affects a success or failure in three-dimensional structure analysis. From this, in the related art, users (experimenters) have needed to perform biochemical experiments and three-dimensional structure analysis on a large number of candidate fusion sequences corresponding to a large number of candidate patterns, which is problematic in terms of time and cost.

Consequently, a sequence extraction process of the sequence extraction system 100 is performed using a target protein as a GPCR, and thus it is possible to extract an amino acid sequence of an inactive GPCR suitable for three-dimensional structure analysis, and it is expected to promote three-dimensional structure analysis of the inactive GPCR.

The target protein may also be, for example, a transporter. In this respect, transporters, like GPCRs, may not be suitable for three-dimensional structure analysis using a cryo-electron microscope. By using the target protein as a transporter, it is possible to extract an amino acid sequence of the transporter suitable for three-dimensional structure analysis, and it is expected to promote three-dimensional structure analysis of the transporter.

In addition, the target protein may be, for example, a protein with a molecular weight of approximately 40 kDa. Thereby, by inserting an insertion polypeptide (particularly, for example, an insertion protein) into the target protein, the protein can be made to have a size suitable for three-dimensional structure analysis, and an amino acid sequence of the protein suitable for three-dimensional structure analysis can be extracted.

Note that the target protein may be any protein.

The insertion polypeptide is, for example, a polypeptide (for example, a protein) to be inserted into the target protein.

Here, the insertion polypeptide may be, for example, a polypeptide of approximately 10 to 20 amino acids. In this case, the insertion polypeptide may be a polypeptide that can be bonded to a predetermined antibody. Thereby, a fusion protein into which the insertion polypeptide has been inserted is bonded to the predetermined antibody, and for example, the overall size of the fusion protein and the predetermined antibody is increased, making it easier to perform three-dimensional structure analysis.

The insertion polypeptide may also be a protein (insertion protein).

The insertion protein may be, for example, T4L (T4 phage lysozyme) or BRIL (cytochrome b562 mutant). In this respect, since T4L and BRIL are insertion proteins that are frequently used in three-dimensional structure analysis, and thus the insertion of the insertion protein may facilitate three-dimensional structure analysis of the fusion protein (ultimately, the target protein).

The insertion protein may also be, for example, a protein whose three-dimensional structure is known, or may be a protein with high thermal stability. The insertion of such an insertion protein may facilitate three-dimensional structure analysis of the fusion protein (ultimately, the target protein).

The insertion protein may also be, for example, a protein having a certain size or larger (for example, a molecular weight equal to or greater than that of the target protein). Thereby, even when the target protein is a small-sized protein, the size of the fusion protein may be increased by the insertion of the insertion protein, making it easier to perform three-dimensional structure analysis.

The insertion polypeptide may also be a polypeptide including an insertion polypeptide (for example, an insertion protein) and a predetermined linker.

The sequence acquisition unit 120 may acquire a plurality of inserted amino acid sequences. Thereby, the candidate sequence generation unit 130 to be described later can generate a candidate fusion sequence into which a plurality of inserted amino acid sequences have been inserted. Note that, in this case, the plurality of inserted amino acid sequences may be inserted amino acid sequences of the same insertion polypeptide, or may be inserted amino acid sequences of different insertion polypeptides.

Note that, for example, the sequence acquisition unit 120 may acquire at least one of target amino acid sequence information and inserted amino acid sequence information from the user terminal 200. The sequence acquisition unit 120 may also acquire at least one of the target amino acid sequence information and the inserted amino acid sequence information from, for example, a database that manages information on amino acid sequences, on the basis of the user's operation of the user terminal 200.

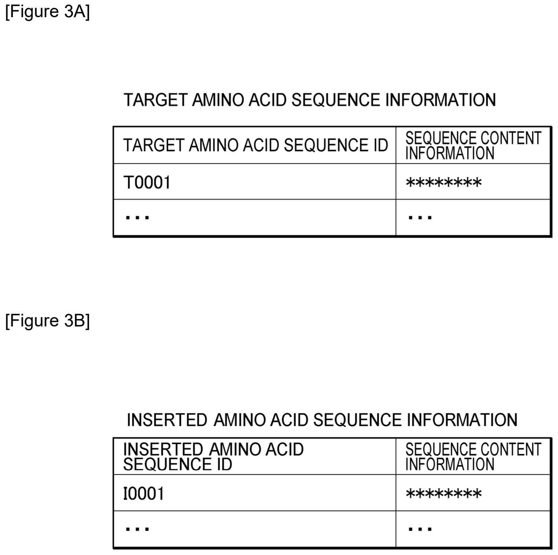

Figure 3A is a diagram showing an example of target amino acid sequence information stored in the storage unit 110. The target amino acid sequence information stored in the storage unit 110 includes, for example, a target amino acid sequence ID and sequence content information.

The target amino acid sequence ID is, for example, information for identifying the target amino acid sequence information stored in the storage unit 110. The sequence content information is, for example, information indicating the content of the amino acid sequences (for example, information in which the type and order of amino acid residues are associated with each other).

Figure 3B is a diagram showing an example of inserted amino acid sequence information stored in the storage unit 110. The inserted amino acid sequence information stored in the storage unit 110 includes, for example, an inserted amino acid sequence ID and sequence content information.

The inserted amino acid sequence ID is information for identifying the inserted amino acid sequence information stored in the storage unit 110.

The candidate sequence generation unit 130 generates a plurality of candidate fusion sequences, which are a plurality of candidates for a fusion sequence in which an inserted amino acid sequence is inserted into the entirety or a part of a target amino acid sequence, on the basis of each of a plurality of candidate insertion patterns which are a plurality of candidates for an insertion pattern for inserting at least one inserted amino acid sequence into the entirety or a part of a target amino acid sequence, and stores candidate fusion sequence information on the candidate fusion sequences in the storage unit 110.

Here, the candidate insertion pattern is not particularly limited, and may be, for example, a pattern in which one or a plurality of inserted amino acid sequences are inserted at a predetermined position in the entire target amino acid sequence (that is, for example, the entire length of an amino acid sequence of a wild-type target protein), or may be, for example, a pattern in which one or a plurality of inserted amino acid sequences are inserted at each of two or more predetermined positions in the entire target amino acid sequence.

In addition, the candidate insertion pattern may be a pattern in which one or a plurality of inserted amino acid sequences are inserted at a predetermined position in a part of the target amino acid sequence (that is, for example, an amino acid sequence in which an amino acid sequence of a wild-type target protein has been subjected to a predetermined modification (for example, mutation, insertion or deletion of an amino acid sequence, or the like)). Alternatively, the candidate insertion pattern may be, for example, a pattern in which one or a plurality of inserted amino acid sequences are inserted at each of two or more predetermined positions in a part of the target amino acid sequence.

In addition, when the target amino acid sequence is a membrane protein (particularly, for example, a GPCR or a transporter), the candidate insertion pattern may be a pattern in which one or a plurality of inserted amino acid sequences are inserted at a predetermined position corresponding to a portion which connects transmembrane helices of the target protein in the target amino acid sequence. In this case, the amino acid sequence corresponding to the portion connecting the transmembrane helices may be appropriately subjected to editing (for example, mutation, insertion or deletion).

In this manner, the candidate sequence generation unit 130 can generate a plurality of candidate fusion sequences corresponding to a plurality of candidate insertion patterns. That is, the candidate sequence generation unit 130 can generate a plurality of candidate fusion sequences that are candidates for selecting (screening) fusion sequences suitable for three-dimensional structure analysis of a target protein.

For example, the candidate sequence generation unit 130 may generate candidate fusion sequences under conditions designated by the user (for example, the number of candidate fusion sequences to be generated, the range of insertion positions, the number of insertion polypeptides, and the like) on the basis of the operation of the user terminal 200 by the user. Thereby, the sequence extraction system 100 can more effectively extract fusion sequences suitable for three-dimensional structure analysis of a target protein.

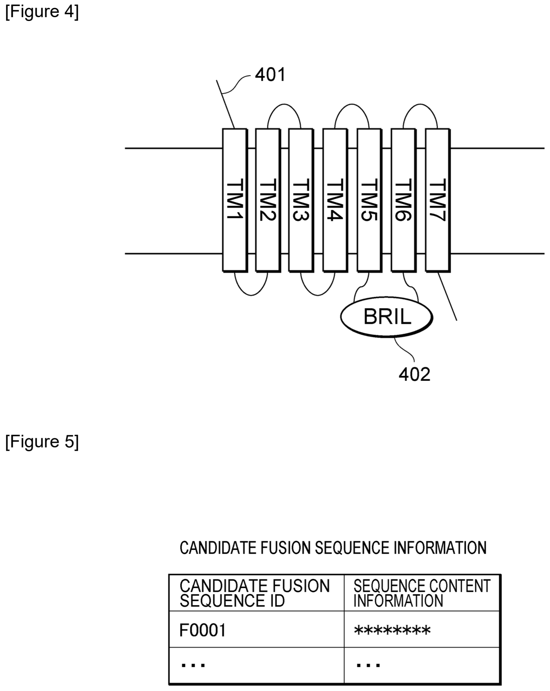

Figure 4 is a diagram showing an example of a candidate insertion pattern. Figure 4 shows an example when a target protein 401 is a membrane protein (7-transmembrane protein) and an insertion polypeptide (in Figure 4, an insertion protein) 402 is BRIL. One insertion polypeptide 402 is inserted at a predetermined position corresponding to a portion connecting transmembrane helices of the target protein 401 (in this case, a portion connecting a transmembrane helix TM5 and a transmembrane helix TM6).

Note that the number and positions at which the insertion polypeptide 402 is inserted are not limited thereto. Furthermore, at the time of insertion, the target amino acid sequence may be appropriately subjected to editing (for example, mutation, insertion or deletion).

Figure 5 is a diagram showing an example of candidate fusion sequence information stored in the storage unit 110. The candidate fusion sequence information stored in the storage unit 110 includes, for example, a candidate fusion sequence ID and sequence content information.

The candidate fusion sequence ID is information for identifying the candidate fusion sequence information stored in the storage unit 110.

The structure prediction result acquisition unit 140 acquires structure prediction result information showing the results of prediction of the structures of a plurality of candidate fusion proteins corresponding to each of a plurality of candidate fusion sequences on the basis of each of the plurality of candidate fusion sequences, and stores the structure prediction result information in the storage unit 110.

The structure prediction result acquisition unit 140 may provide, for example, a plurality of pieces of corresponding candidate fusion sequence information corresponding to the plurality of candidate fusion sequences to a predetermined functional unit (for example, a structure prediction unit) included in the sequence extraction system 100 to acquire structure prediction result information from the predetermined functional unit.

The structure prediction result acquisition unit 140 may also provide, for example, a plurality of pieces of candidate fusion sequence information corresponding to the plurality of candidate fusion sequences to the structure prediction system 300 to acquire structure prediction result information from the structure prediction system 300.

Here, the structure prediction result information is, for example, information indicating a predicted structure of a candidate fusion protein corresponding to each of the plurality of candidate fusion sequences (that is, for example, information indicating the coordinates of each amino acid residue).

The structure prediction result information may further include, for example, higher-order structure information indicating a higher-order structure (secondary structure, tertiary structure, or quaternary structure) formed by each amino acid residue on the basis of each amino acid residue. That is, the higher-order structure formed by each amino acid residue can be grasped with reference to the higher-order structure information.

The structure prediction result information may further include, for example, bonding information for each amino acid residue, the bonding information including an amino acid residue (that is, an amino acid residue that forms a bonding pair) to which each amino acid residue is bonded, as well as bonding information indicating the mode of coupling. That is, it is possible to grasp the amino acid residue that forms a bonding pair for each amino acid residue with reference to the bonding information. Note that, here, the bonding may be, for example, a covalent bond, a peptide bond, a disulfide bond, an ionic bond, a hydrogen bond, or a hydrophobic interaction.

Here, the structure prediction result information may be refined on the basis of, for example, processing of a known structure prediction program. The known structure prediction program may be, for example, AlphaFold2 (Jumper, J., Evans, R., Pritzel, A. et al. Highly accurate protein structure prediction with AlphaFold. Nature 596, 583-589 (2021)).

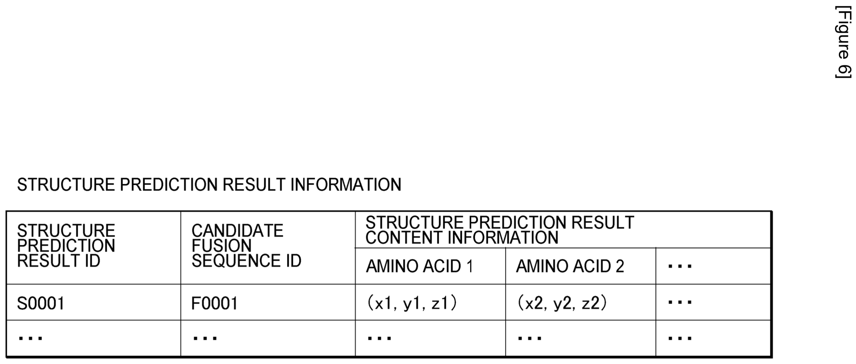

Figure 6 is a diagram showing an example of structure prediction result information stored in the storage unit 110. The structure prediction result information stored in the storage unit 110 includes, for example, a structure prediction result **ID,** a candidate fusion sequence **ID,** and structure prediction result content information.

The structure prediction result **ID** is information for identifying the structure prediction result information stored in the storage unit 110.

The structure prediction result content information is information indicating the content of a structure prediction result, for example, information indicating the coordinates (for example, an X coordinate, a Y coordinate, and a Z coordinate) of each amino acid residue of a candidate fusion sequence.

The evaluation unit 150 evaluates an insertion site where an inserted amino acid sequence is inserted, on the basis of the prediction result (structure prediction result information) obtained by the structure prediction result acquisition unit 140, and stores evaluation result information on the evaluation result in the storage unit 110.

Specifically, for example, the evaluation unit 150 evaluates an insertion site (particularly, for example, the flexibility of the insertion site) to evaluate whether a variation in the orientation of a target protein and an insertion polypeptide in a candidate fusion protein is limited.

In this regard, when a variation in the orientation of the target protein and the insertion polypeptide is diverse, a fusion protein takes various structures corresponding to the variation, and the structure of the fusion protein constantly fluctuates. For this reason, it may be difficult to crystallize the fusion protein, and it may be difficult to analyze images captured by an electron microscope (for example, 3D reconstruction of images, and the like).

On the other hand, when the variation in the orientation of the target protein and the insertion polypeptide in the candidate fusion protein is limited, a variation in the structure of the candidate fusion protein is limited. For this reason, a candidate fusion sequence may be advantageous for three-dimensional structure analysis of the candidate fusion protein. Consequently, the evaluation unit 150 evaluates, for example, an insertion site (particularly, for example, the flexibility of the insertion site) to evaluate whether the variation in the orientation is limited.

The evaluation unit 150 can evaluate whether the insertion site contributes to the formation of a secondary structure of a protein on the basis of the prediction result (structure prediction result information) obtained by the structure prediction result acquisition unit 140. Here, the secondary structure may be, for example, an α-helix structure, or a 3₁₀ helix structure, a π-helix structure, a β-sheet structure, a β-turn structure, or an ω-loop structure.

When the insertion site forms a secondary structure, it is considered that the target protein and the insertion polypeptide form a series of secondary structures in the vicinity of the insertion site. In this case, the variation in the orientation of the target protein and the insertion polypeptide in the candidate fusion protein may be limited. For this reason, the evaluation unit 150 evaluates whether the insertion site contributes to the formation of a secondary structure of a protein, thereby evaluating whether the variation in the orientation of the target protein and the insertion polypeptide in the candidate fusion protein is limited.

At this time, when the structure prediction result information includes higher-order structure information, the evaluation unit 150 may evaluate whether the insertion site contributes to the formation of a secondary structure of a protein on the basis of the structure prediction result information (particularly, the higher-order structure information).

In addition, the evaluation unit 150 can evaluate whether the insertion site contributes to the formation of a secondary structure in accordance with a pattern of hydrogen bonds predicted on the basis of the coordinates of atoms in a structure to be predicted.

The secondary structure of the protein is formed through hydrogen bonds (particularly, for example, hydrogen bonds between amide hydrogen atoms and carbonyl oxygen atoms in a peptide backbone). The type of secondary structure can be determined on the basis of the pattern of hydrogen bonds. Specifically, for example, in the case of an α-helix structure, an amide hydrogen atom forms a hydrogen bond with a carbonyl oxygen atom that is four residues apart.

Consequently, the evaluation unit 150 can predict the pattern of hydrogen bonds to be formed on the basis of the structure prediction result information, and can evaluate whether the insertion site contributes to the formation of a secondary structure in accordance with the predicted pattern of hydrogen bonds.

At this time, the evaluation unit 150 may predict that a hydrogen bond will be formed between two atoms when a distance between the two atoms is within a predetermined distance, for example, on the basis of the coordinates of the amide hydrogen atom and the coordinates of the carbonyl oxygen atom.

Furthermore, when the structure prediction result information includes bonding information, the evaluation unit 150 may evaluate whether the insertion site contributes to the formation of a secondary structure of a protein on the basis of the structure prediction result information (particularly, bonding information).

The evaluation unit 150 can also evaluate the insertion site on the basis of a score (predicted aligned error (PAE) score) regarding an error of each prediction result of each of a plurality of atoms included in the predicted structure indicated by the structure prediction result information, relative to each of the prediction result of each of the plurality of atoms.

Here, the PAE score will be described.

In the related art, a PAE score is known as an index for evaluating the reliability of a structure prediction result between domains in a protein structure configured with a plurality of domains. That is, for example, when a PAE score in two atoms belonging to different domains is low (small value), a predicted structure regarding the orientation of the two domains to which the two atoms belong is evaluated as being reliable.

Specifically, a plurality of atoms included in a predicted structure indicated by structure prediction result information are assumed to be, for example, a first atom, a second atom, and a third atom. A PAE score of the first atom includes a PAE score for a prediction result of the second atom (in other words, a PAE score of the first atom with respect to the second atom) and a PAE score for a prediction result of the third atom (in other words, a PAE score of the first atom with respect to the third atom). The PAE score includes the PAE score of the first atom, the PAE score of the second atom, and the PAE score of the third atom.

The PAE score of the first atom with respect to the second atom indicates the reliability of the prediction result of the first atom, for example, if the prediction result of the second atom is correct. For example, when the PAE score of the first atom with respect to the second atom is low, it indicates that the reliability of the prediction result of the first atom is high when the prediction result of the second atom is correct, and an error of the prediction result is small. The same is true of the PAE score of the first atom with respect to the third atom.

In other words, when the first and second atoms belong to different domains (a first domain and a second domain), the reliability of the prediction result of the first atom when the prediction result of the second domain is correct indicates the reliability of a prediction result of the first domain when the prediction result of the second domain is correct. When the reliability of the prediction result of the first domain when the prediction result of the second domain is correct is low, it can be evaluated that a prediction result of the orientation of the first domain and the second domain is low and a variation in the orientation is diverse.

That is, a PAE score can be low, for example, when the variation in the orientation between the domain to which the first atom belongs and the domain to which the second atom belongs is limited. In addition, a PAE score can be high when the variation in the orientation between the domain to which the first atom belongs and the domain to which the second atom belongs is diverse.

Here, the PAE score may be calculated on the basis of, for example, a feature value related to a positional relationship and distance between amino acid residues in each of a plurality of candidate fusion sequences in a known structure prediction program.

Note that the feature value related to the positional relationship and distance between amino acid residues in the known structure prediction program may be calculated, for example, by performing a predetermined sequence analysis on an amino acid sequence of a protein (for example, a homologous protein (or a paralogous protein, an orthologous protein, or an analogous protein)) that is referred to for structure prediction of a target protein in the known structure prediction program.

In this manner, a PAE score (that is, a PAE score for each of a plurality of atoms included in a predicted structure, relative to each of the plurality of atoms) can be said to be information indicating an error between a prediction result of the structure and a correct structure of a protein that is a target for prediction. In this regard, when considering a fusion sequence to be subjected to three-dimensional structure analysis, a correct structure (for example, a correct structure of a target protein or a fusion protein) is unknown, and thus a PAE score can be an index of the reliability of a prediction result of the structure.

That is, the evaluation unit 150 can evaluate that, when the PAE score related to the error is equal to or less than a predetermined threshold value on the basis of the PAE score, a variation in the orientation between a target protein and an insertion polypeptide is limited.

When an insertion site is evaluated as an amino acid sequence suitable for three-dimensional structure analysis on the basis of the results of the evaluation, the evaluation unit 150 can evaluate that the insertion site is "suitable". That is, the evaluation unit 150 can evaluate that the insertion site is "suitable", for example, when it is evaluated that the insertion site contributes to the formation of a secondary structure of a protein or when the PAE score is equal to or less than a predetermined threshold value.

In addition, when it is evaluated that the insertion site contributes to the formation of a secondary structure of a protein and the PAE score is equal to or less than a predetermined threshold value, the evaluation unit 150 can evaluate that the insertion site is "suitable".

That is, the evaluation unit 150 can evaluate whether the insertion site contributes to the formation of a secondary structure of a protein further on the basis of the PAE score. Thereby, the evaluation unit 150 can improve the accuracy of the evaluation (secondary structure evaluation) regarding whether the insertion site contributes to the formation of a secondary structure of a protein further on the basis of the PAE score.

When the evaluation unit 150 evaluates an insertion site into which an inserted amino acid sequence is inserted on the basis of a prediction result (structure prediction result information), the evaluation unit 150 can first evaluate whether the insertion site contributes to the formation of a secondary structure of a protein on the basis of a prediction result (structure prediction result information) obtained by the structure prediction result acquisition unit 140, and then evaluate the insertion site on the basis of the PAE score. That is, the evaluation unit 150 can evaluate the insertion site on the basis of two indices, that is, whether the secondary structure is formed and the PAE score. Thereby, the sequence extraction unit 160, which will be described later, can extract appropriate candidate fusion sequences that satisfy the criteria of the two indices.

When the evaluation unit 150 evaluates whether the insertion site contributes to the formation of a secondary structure of a protein on the basis of the prediction result (structure prediction result information), and when a structure to be predicted is inaccurate, the evaluation of the evaluation unit 150 may be inappropriate. Consequently, the evaluation unit 150 can further evaluate the insertion site on the basis of the PAE score, thereby evaluating whether the insertion site contributes to the formation of a secondary structure in a predicted structure with a certain degree of accuracy.

Note that the order and combination of evaluations of the evaluation unit 150 are not limited to the above. For example, first, the insertion site may be evaluated on the basis of the PAE score, and further, it may be evaluated whether the insertion site contributes to the formation of a secondary structure of a protein on the basis of the prediction result (structure prediction result information) obtained by the structure prediction result acquisition unit 140.

As described above, in the related art, a PAE score is known as an index for evaluating the reliability of a structure prediction result between domains in a protein structure configured with a plurality of domains. That is, when a PAE score in two atoms belonging to different domains is low, a predicted structure regarding the orientation of the two domains to which the two atoms belong is evaluated as being reliable.

However, the evaluation unit 150 can use a PAE score for secondary structure evaluation. That is, in this case, the evaluation unit 150 does not use the PAE score to evaluate the reliability of a prediction result between domains, but uses the PAE score to evaluate whether a target protein and an insertion polypeptide form a series of secondary structures in the vicinity of an insertion site, and uses the PAE score for a purpose different from the purpose of using a PAE score which is known in the related art.

Note that a PAE score has been described as a score in which a predicted structure regarding the orientation of two domains to which two atoms belong is evaluated as being reliable when the score is low (small value), but it may also be a score in which the predicted structure regarding the orientation of the two domains to which the two atoms belong is evaluated as being reliable when the score is high (large value). In this case, the evaluation unit 150 may evaluate that an insertion site is "suitable", for example, when it is evaluated that the insertion site contributes to the formation of a secondary structure of a protein or when the PAE score is equal to or greater than a predetermined threshold value.

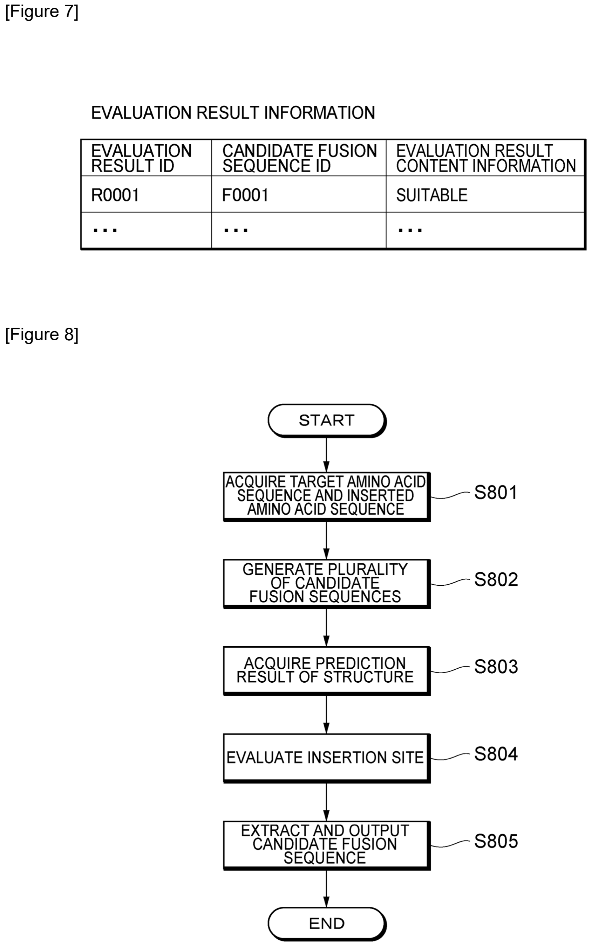

Figure 7 is a diagram showing an example of evaluation result information stored in the storage unit 110. The evaluation result information stored in the storage unit 110 includes, for example, an evaluation result ID, a candidate fusion sequence ID, and evaluation result content information.

The evaluation result ID is information for identifying the evaluation result information stored in the storage unit 110.

The evaluation result content information is information indicating the result of the evaluation of the evaluation unit 150. The evaluation result content information may include, for example, "suitable" indicating that an insertion site is evaluated as a target to be extracted by the sequence extraction unit 160 to be described later and is suitable for three-dimensional structure analysis.

The sequence extraction unit 160 extracts at least one candidate fusion sequence from a plurality of candidate fusion sequences on the basis of the result of the evaluation of the evaluation unit 150.

Specifically, the sequence extraction unit 160 can extract at least one candidate fusion sequence that is evaluated as being "suitable" by the evaluation unit 150.

That is, the sequence extraction unit 160 can extract, for example, at least one candidate fusion sequence in which an insertion site is evaluated as forming a secondary structure of a protein, at least one candidate fusion sequence whose PAE score is equal to or less than a predetermined threshold value, or at least one candidate fusion sequence in which an insertion site is evaluated as forming a secondary structure of a protein and a PAE score is equal to or less than a predetermined threshold value.

The output unit 170 outputs at least one candidate fusion sequence extracted by the sequence extraction unit 160 to the user terminal 200. Thereby, the user can acquire candidate fusion sequences and, for example, perform three-dimensional structure analysis using the candidate fusion sequences.

Subsequently, a first specific example of a sequence extraction process using the sequence extraction system 100 will be described.

The sequence acquisition unit 120 acquired, from the user terminal 200, target amino acid sequence information in which a target protein is arginine vasopressin receptor 2 (AVPR2), and inserted amino acid sequence information in which an insertion polypeptide (in this case, an insertion protein) is BRIL. Here, AVPR2 is a GPCR. The candidate sequence generation unit 130 generated 100 types of candidate fusion sequences by inserting BRIL into the arginine vasopressin receptor 2 (AVPR2). The structure prediction result acquisition unit 140 acquired structure prediction result information showing results of prediction of the respective structures of 100 types of candidate fusion proteins respectively corresponding to the 100 types of candidate fusion sequences.

Then, the evaluation unit 150 evaluated whether an insertion site contributes to the formation of a secondary structure of a protein on the basis of the structure prediction result information, and specified six types of candidate fusion sequences. Further, the evaluation unit 150 evaluated insertion sites on the basis of PAE scores in the six types of candidate fusion sequences, and specified three types of candidate fusion sequences whose PAE scores are equal to or less than a predetermined threshold value. The sequence extraction unit 160 extracted the three types of candidate fusion sequences, and the output unit 170 outputted the three types of candidate fusion sequences to the user terminal 200.

Small-scale biochemical experiments (for example, expression and purification of a protein, and addition of tolvaptan (antagonist)) were performed using the three types of candidate fusion sequences, and the most appropriate candidate fusion sequence was selected from among the three types. Then, large-scale biochemical experiments (for example, expression and purification of a protein, and addition of tolvaptan (antagonist)) were performed using the one type of candidate fusion sequence, and the structure of tolvaptan-coupled AVPR2 (that is, an inactive GPCR to which an antagonist is bonded) was successfully determined by cryo-electron microscopy.

In addition, a large-scale biochemical experiment (for example, expression and purification of a protein, and addition of an agonist) was performed using the one type of candidate fusion sequence, and the structure of an agonist-coupled AVPR2 was successfully determined by cryo-electron microscopy.

Next, a second specific example of a sequence extraction process using the sequence extraction system 100 will be described.

The sequence acquisition unit 120 acquired, from the user terminal 200, target amino acid sequence information in which a target protein is bradykinin receptor 2 (BDKRB2), and inserted amino acid sequence information in which an insertion polypeptide (in this case, an insertion protein) is BRIL. Here, BDKRB2 is a GPCR. The candidate sequence generation unit 130 generated 100 types of candidate fusion sequences by inserting BRIL into the bradykinin receptor 2 (BDKRB2). The structure prediction result acquisition unit 140 acquired structure prediction result information showing results of prediction of the structures of 100 types of candidate fusion proteins respectively corresponding to the 100 types of candidate fusion sequences.

Then, the evaluation unit 150 evaluated whether an insertion site contributes to the formation of a secondary structure of a protein on the basis of the structure prediction result information, and specified 36 types of candidate fusion sequences. Furthermore, the evaluation unit 150 evaluated an insertion site on the basis of PAE scores in the 36 types of candidate fusion sequences, and specified seven types of candidate fusion sequences whose PAE scores are equal to or less than a predetermined threshold value. The sequence extraction unit 160 extracted the seven types of candidate fusion sequences, and the output unit 170 outputted the seven types of candidate fusion sequences to the user terminal 200.

Small-scale biochemical experiments (for example, expression and purification of a protein, and addition of icatibant (antagonist)) were performed using the seven types of candidate fusion sequences, and the most appropriate candidate fusion sequence was selected from among the seven types. Then, large-scale biochemical experiments (for example, expression and purification of a protein, and addition of icatibant (antagonist)) were performed using the one type of candidate fusion sequence, and the structure of icatibant-coupled BDKRB2 (that is, an inactive GPCR to which an antagonist is bonded) was successfully determined by cryo-electron microscopy.

In X-ray crystal structure analysis of the related art and structure analysis of the related art using a cryo-electron microscope, it takes a huge amount of time to screen for an amino acid sequence suitable for crystallization. In the case of the X-ray crystal structure analysis, it takes several years to 10 years to determine the structure. In the case of the structure analysis using a cryo-electron microscope, it takes several months to a year to determine the structure.

However, in this specific example using the sequence extraction system 100, the extraction of candidate fusion sequences is completed in approximately 10 to 20 hours, and the structure is determined in approximately one month. In this manner, the sequence extraction system 100 can significantly reduce the time and cost required to determine the structure.

Figure 8 is a flowchart showing an example of processing in the sequence extraction system 100.

First, the sequence acquisition unit 120 acquires a target amino acid sequence and an inserted amino acid sequence (S801). The candidate sequence generation unit 130 generates a plurality of candidate fusion sequences on the basis of each of a plurality of insertion patterns (S802).

The structure prediction result acquisition unit 140 acquires structure prediction result information indicating the results of prediction of the structure of each of the plurality of candidate fusion sequences (S803). The evaluation unit 150 evaluates an insertion site on the basis of the prediction results (S804).

Then, the sequence extraction unit 160 extracts at least one candidate fusion sequence from the plurality of candidate fusion sequences on the basis of the evaluation results, and the output unit 170 outputs the extracted at least one candidate fusion sequence to the user terminal 200 (S805).

### <Modification Example>

Next, a modification example of the sequence extraction system 100 will be described.

The evaluation unit 150 can evaluate an insertion site on the basis of an angle determined by the orientation of first and second helices of a target protein which are connected to an insertion polypeptide.

Here, in the candidate fusion sequence, for example, the first helix, the insertion polypeptide, and the second helix are connected to each other. That is, for example, in the candidate fusion sequence, the insertion polypeptide is inserted between the first and second helices of the target protein.

Specifically, description will be given on the basis of an example of a candidate insertion pattern shown in Figure 4. In the example shown in Figure 4, an insertion polypeptide (in this case, BRIL) is inserted into a part that connects the transmembrane helix TM5 and the transmembrane helix TM6. That is, TM5 and TM6 are first and second helices of a target protein which are connected to the insertion polypeptide. Note that it does not matter what helices the first and second helices are, or whether the first and second helices are transmembrane helices.

An angle determined by the orientation of the first and second helices includes, for example, a first angle centered on an intersection between the axis of the first helix and a first plane which is perpendicular to the axis of the first helix to which a first atom (for example, a Cα atom) of an amino acid residue at an insertion site belongs, and a second angle centered on an intersection between the axis of the second helix and a second plane which is perpendicular to the axis of the second helix and to which a second atom (for example, a Cα atom) of an amino acid residue at the insertion site belongs. More specifically, the first angle may be, for example, an angle determined by the first atom and the intersection between the first plane and the axis of the second helix with an intersection between the axis of the first helix and the first plane as the center. In addition, the second angle may be, for example, an angle determined by the second atom and an intersection between the second plane and the axis of the first helix with the intersection between the axis of the second helix and the second plane as the center.

The axis of a helix is determined by, for example, a predetermined formula on the basis of the coordinates of a plurality of amino acid residues belonging to the helix (particularly, for example, the coordinates of a Cα atom).

The plurality of amino acid residues belonging to the helix may be, for example, amino acid residues belonging to a predetermined range designated in advance by the administrator of the sequence extraction system 100. The predetermined range may include, for example, amino acid residues in the helix from an amino acid residue, which is conserved in the family to which the target protein belongs to, to the insertion site. When the target protein is a GPCR, the conserved amino acid residues may be proline in TM5 (5.50 in Ballesteros-Weinstein numbering notation) and proline in TM6 (6.50 in Ballesteros-Weinstein numbering notation).

The first atom and the second atom are, for example, atoms of an amino acid residue at the boundary of the insertion site. The first atom is, for example, an atom of an amino acid residue at the boundary of the insertion site between the first helix and the insertion polypeptide, and the second atom is, for example, an atom of an amino acid residue at the boundary of the insertion site between the second helix and the insertion polypeptide. The first atom and the second atom may be atoms of an amino acid residue of a target protein, or may be atoms of an amino acid residue of an insertion polypeptide. In addition, the first atom and the second atom may be atoms of an amino acid residue of a protein (for example, an adenosine A2A receptor or a de novo protein) different from the target protein and the insertion polypeptide which is inserted during insertion of the insertion polypeptide.

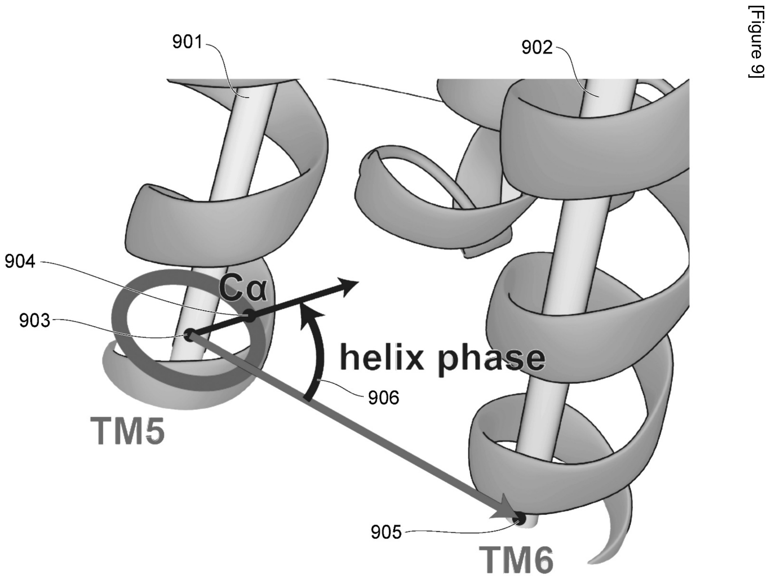

A more specific description will be given using Figure 9. Figure 9 is a diagram showing an example of a first angle. An axis 901 is, for example, the axis of a first helix (in this case, TM5). An axis 902 is, for example, the axis of a second helix (in this case, TM6). A point 903 is, for example, an intersection between the axis of the first helix and a first plane. A point 904 is, for example, a first atom. A point 905 is, for example, an intersection between the first plane and the axis 902. An angle 906 is a first angle, and is, for example, an angle determined by the points 904 and 905 with the point 903 as the center. Note that a second angle is calculated in the same manner.

For example, when the first angle is within a predetermined first angle range, the evaluation unit 150 can evaluate that the insertion site is "suitable". In addition, for example, when the second angle is within a predetermined second angle range, the evaluation unit 150 can evaluate that the insertion site is "suitable". In addition, when the first angle is within the predetermined first angle range and the second angle is within the predetermined second angle range, the evaluation unit 150 can evaluate that the insertion site is "suitable". Thereby, the sequence extraction system 100 can easily extract an amino acid sequence of a protein suitable for three-dimensional structure analysis, in consideration of the first angle and the second angle.

In addition, when satisfying at least one of a case where it is evaluated that the insertion site contributes to the formation of a secondary structure of a protein and a case where it is evaluated that a PAE score is equal to or less than a predetermined threshold value, the evaluation unit 150 can further evaluate at least one of whether the first angle is within the predetermined first angle range and whether the second angle is within the predetermined second angle range and can evaluate that the insertion site is "suitable". Thereby, for example, when candidate fusion sequences are searched for in a wide range on the basis of at least one of the evaluation that the insertion site contributes to the formation of a secondary structure of a protein and the evaluation that the PAE score is equal to or less than a predetermined threshold value, the sequence extraction system 100 can easily extract an amino acid sequence of a protein suitable for three-dimensional structure analysis by further considering at least one of the first angle and the second angle even when the number of sequences to be extracted is still equal to or greater than a certain number.

Note that preferred first and second angle ranges differ depending on the type of an insertion polypeptide and an insertion site of the insertion polypeptide. The first and second angle ranges may be values that are set in advance by the administrator of the sequence extraction system 100, for example. In this case, the first and second angle ranges may be set, for example, for each type of insertion polypeptide or for each insertion site of an insertion polypeptide.

In this regard, when at least one of the first angle and the second angle in a candidate fusion sequence is not within the first angle range and the second angle range, the structure of a fusion protein corresponding to the candidate fusion sequence is predicted to have a predetermined structure by calculation, but in reality, the expression level may be drastically reduced or may not be expressed at all. As a cause, when at least one of the first angle and the second angle is not within the first angle range and the second angle range, for example, an inappropriate load may be applied to a helix at or near an insertion site, thereby hindering the formation of appropriate folding. For this reason, the evaluation unit 150 evaluates an insertion site on the basis of at least one of the first angle and the second angle, and thus it is possible to easily extract an amino acid sequence that does not apply an inappropriate load to an insertion site and a helix near the insertion site, particularly an amino acid sequence of a fusion protein capable of obtaining a certain expression level, as an amino acid sequence of a protein suitable for three-dimensional structure analysis.

The evaluation unit 150 can evaluate an insertion site on the basis of a distance between the insertion site and a lipid bilayer predicted when a target protein is embedded in the lipid bilayer. In this case, the evaluation unit 150 can evaluate the insertion site on the basis of a first distance indicating a distance between a first atom (for example, a Cα atom) and the lipid bilayer, and a second distance indicating a distance between a second atom (for example, a Cα atom) and the lipid bilayer.

The first atom and the second atom are, for example, atoms of an amino acid residue at the boundary of the insertion site. The first atom is, for example, an atom of the amino acid residue at the boundary of the insertion site between a first helix and an insertion polypeptide, and the second atom is, for example, an atom of the amino acid residue at the boundary of the insertion site between a second helix and the insertion polypeptide. The first atom and the second atom may be atoms of an amino acid residue of a target protein, or may be atoms of an amino acid residue of an insertion polypeptide. The first atom and the second atom may also be atoms of an amino acid residue of a protein (for example, an adenosine A2A receptor or a de novo protein) that is different from the target protein and the insertion polypeptide and that is inserted during the insertion of the insertion polypeptide.

The target protein may be, for example, a membrane protein. The position of the lipid bilayer may be, for example, the position of the lipid bilayer which is calculated by a membrane position prediction program for predicting the position of the lipid bilayer when the membrane protein is embedded in the lipid bilayer, on the basis of the amino acid sequence or structure of the membrane protein. The membrane position program can predict the position of the lipid bilayer in accordance with, for example, the properties (for example, polarity) of amino acid residues included in the helix of the membrane protein. Any membrane position prediction program may be used, and may be, for example, orientation and evaluation of membrane proteins (OREMPRO).

The first distance and the second distance may each be, for example, a perpendicular distance between the atom (the first atom and the second atom) of the amino acid residue located at the boundary of the insertion site and a layer surface formed by the lipid bilayer.

For example, when the first distance is within a predetermined first distance range (for example, a predetermined distance or more), the evaluation unit 150 can evaluate that the insertion site is "suitable". In addition, for example, when the second distance is within a predetermined second distance range (for example, a predetermined distance or more), the evaluation unit 150 can evaluate that the insertion site is "suitable". In addition, when the first distance is within the predetermined first distance range and the second distance is within the predetermined second distance range, the evaluation unit 150 can evaluate that the insertion site is "suitable". Thereby, the sequence extraction system 100 can easily extract an amino acid sequence of a protein suitable for three-dimensional structure analysis, in consideration of the first distance and the second distance.

In addition, when satisfying at least one of a case where it is evaluated that the insertion site contributes to the formation of a secondary structure of a protein and a case where it is evaluated that a PAE score is equal to or less than a predetermined threshold value, the evaluation unit 150 can further evaluate at least one of whether the first distance is within the predetermined first distance range and whether the second distance is within the predetermined second distance range and can evaluate that the insertion site is "suitable". Thereby, for example, when candidate fusion sequences are searched for in a wide range on the basis of at least one of the evaluation that the insertion site contributes to the formation of a secondary structure of a protein and the evaluation that the PAE score is equal to or less than a predetermined threshold value, the sequence extraction system 100 can easily extract an amino acid sequence of a protein suitable for three-dimensional structure analysis by further considering at least one of the first distance and the second distance even when the number of sequences to be extracted is still equal to or greater than a certain number.

Note that preferred first and second distance ranges differ depending on the type of an insertion polypeptide and an insertion site of the insertion polypeptide. The first and second distance ranges may be values that are set in advance by the administrator of the sequence extraction system 100, for example. In this case, the first and second distance ranges may be set, for example, for each type of insertion polypeptide or for each insertion site of an insertion polypeptide.

In this regard, when at least one of the first distance and the second distance in a candidate fusion sequence is not within the first distance range and the second distance range, the structure of a fusion protein corresponding to the candidate fusion sequence is predicted to have a predetermined structure by calculation, but in reality, the expression level may be drastically reduced or may not be expressed at all. As a cause, when at least one of the first distance and the second distance is not within the first distance range and the second distance range, for example, appropriate folding may not be formed. One factor for this is that the insertion polypeptide is a soluble protein (water-soluble protein). For this reason, the evaluation unit 150 evaluates an insertion site on the basis of at least one of the first distance and the second distance, and thus it is possible to easily extract an amino acid sequence of a fusion protein that provides a certain expression level, as an amino acid sequence of a protein suitable for three-dimensional structure analysis.

In addition, when at least one of the first distance and the second distance is not within the first distance range and the second distance range, more specifically, when a distance between the insertion polypeptide and the lipid bilayer is smaller than a predetermined threshold value, the candidate sequence generation unit 130 can generate a candidate fusion sequence in which a third amino acid sequence different from the target amino acid sequence and the inserted amino acid sequence is inserted between the target amino acid sequence and the inserted amino acid sequence when generating a plurality of candidate fusion sequences.

Here, the third amino acid sequence may be, for example, an artificially designed amino acid sequence, an amino acid sequence of an artificially designed de novo protein, or an amino acid sequence of a protein of a family to which the target protein belongs (for example, an adenosine A2A receptor).

The evaluation unit 150 may also evaluate an insertion site by evaluating whether the insertion site contributes to the formation of a secondary structure of a protein, performing evaluation on the basis of a PAE score, evaluating at least one of whether the first angle is within the predetermined first angle range and whether the second angle is within the predetermined second angle range, and evaluating at least one of whether the first distance is within the predetermined first distance range and whether the second distance is within the predetermined second distance range.

For example, the candidate sequence generation unit 130 can generate a candidate fusion sequence by inserting the third amino acid sequence so as to supplement the target amino acid sequence deleted when inserting the insertion polypeptide, on the basis of the amino acid sequence conserved in the family to which the target protein belongs. At this time, the candidate sequence generation unit 130 may supplement the third amino acid sequence corresponding to the deleted target amino acid sequence, on the basis of the amino acid sequence conserved in the family to which the target protein belongs.

The candidate sequence generation unit 130 generates a candidate fusion sequence by inserting the third amino acid sequence between the target amino acid sequence and the inserted amino acid sequence, and thus a distance between an insertion site and a predicted lipid bilayer, which will be described later, is extended, and at least one of the first distance and the second distance falls within the first distance range and the second distance range. Thus, the sequence extraction system 100 can easily extract an amino acid sequence of a protein suitable for three-dimensional structure analysis.

Subsequently, a third specific example of a sequence extraction process using the sequence extraction system 100 will be described.

The sequence acquisition unit 120 acquires, from the user terminal 200, target amino acid sequence information in which a target protein is bradykinin receptor 2 (BDKRB2), and inserted amino acid sequence information in which an insertion polypeptide (in this case, an insertion protein) is BRIL. Here, BDKRB2 is a GPCR. The candidate sequence generation unit 130 generates 100 types of candidate fusion sequences by inserting BRIL into the bradykinin receptor 2 (BDKRB2). The structure prediction result acquisition unit 140 acquires structure prediction result information showing results of prediction of the structures of 100 types of candidate fusion proteins respectively corresponding to the 100 types of candidate fusion sequences.

Then, the evaluation unit 150 evaluates whether an insertion site contributes to the formation of a secondary structure of a protein on the basis of the structure prediction result information, and specifies 32 types of candidate fusion sequences. Furthermore, the evaluation unit 150 evaluates an insertion site on the basis of PAE scores in the 32 types of candidate fusion sequences, and specifies 13 types of candidate fusion sequences whose PAE scores are equal to or less than a predetermined threshold value.

The evaluation unit 150 further evaluates an insertion site on the basis of the first angle and the second angle, and specifies four types of candidate fusion sequences.

The sequence extraction unit 160 extracts the four types of candidate fusion sequences, and the output unit 170 outputs the four types of candidate fusion sequences to the user terminal 200.

Small-scale biochemical experiments (for example, expression and purification of a protein, and addition of icatibant (antagonist)) are performed using the four types of candidate fusion sequences, and the most appropriate one type of candidate fusion sequence is selected from among the four types. Then, large-scale biochemical experiments (for example, expression and purification of a protein, and addition of icatibant (antagonist)) are performed using the one type of candidate fusion sequence, and the structure of icatibant-coupled BDKRB2 (that is, an inactive GPCR to which the antagonist is bonded) is successfully determined by cryo-electron microscopy.

Subsequently, a fourth specific example of a sequence extraction process using the sequence extraction system 100 will be described.

The sequence acquisition unit 120 acquires, from the user terminal 200, target amino acid sequence information in which a target protein is OPN5, which is a non-visual photoreceptor, and inserted amino acid sequence information in which an insertion polypeptide (in this case, an insertion protein) is BRIL. Here, OPN5 is a GPCR. The candidate sequence generation unit 130 generates 196 types of candidate fusion sequences by inserting BRIL into OPN5. The candidate sequence generation unit 130 generates 196 types of candidate fusion sequences including a candidate fusion sequence in which a third amino acid sequence (in this case, an adenosine A2A receptor) is inserted between a target amino acid sequence and an inserted amino acid sequence. The structure prediction result acquisition unit 140 acquires structure prediction result information showing results of prediction of the respective structures of 196 types of candidate fusion proteins respectively corresponding to the 196 types of candidate fusion sequences.

Then, the evaluation unit 150 evaluates whether an insertion site contributes to the formation of a secondary structure of a protein on the basis of the structure prediction result information, and specifies 72 types of candidate fusion sequences. Further, the evaluation unit 150 evaluates insertion sites on the basis of PAE scores in the 72 types of candidate fusion sequences, and specifies 45 types of candidate fusion sequences whose PAE scores are equal to or less than a predetermined threshold value.

Furthermore, the evaluation unit 150 evaluates an insertion site on the basis of a distance between the insertion site and the lipid bilayer predicted when a target protein is embedded in the lipid bilayer, and specifies 45 types of candidate fusion sequences. The evaluation unit 150 also evaluates an insertion site on the basis of the first angle and the second angle, and specifies four types of candidate fusion sequences.

The sequence extraction unit 160 extracts the four types of candidate fusion sequences, and the output unit 170 outputs the four types of candidate fusion sequences to the user terminal 200.

Small-scale biochemical experiments (for example, expression and purification of a protein, and addition of 11-cis-retinal) are performed using the four types of candidate fusion sequences, and the most appropriate one type of candidate fusion sequence is selected from among the four types. Then, large-scale biochemical experiments (for example, expression and purification of a protein, and addition of 11-cis-retinal) are performed using the one type of candidate fusion sequence, and the structure of 11-cis-retinal-coupled OPN5 (that is, an inactive GPCR) is successfully determined by cryo-electron microscopy.

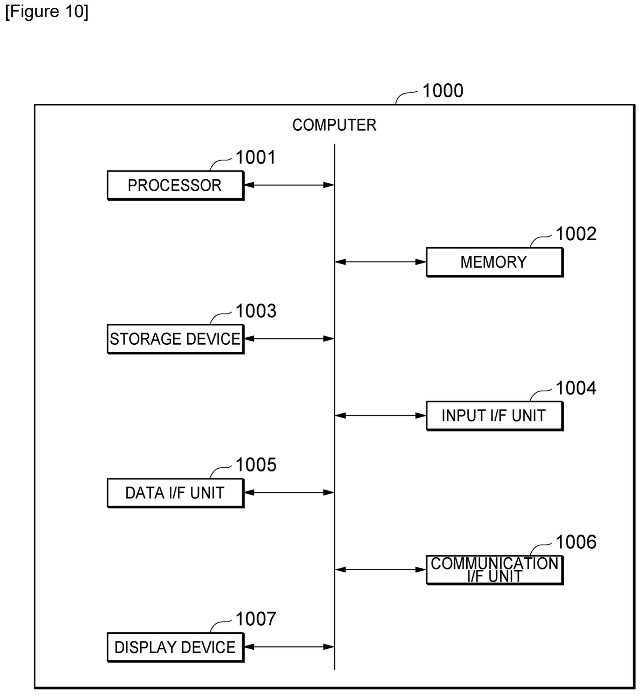

Next, an example of a hardware configuration when the sequence extraction system 100 is implemented by a computer 1000 will be described with reference to Figure 10. Figure 10 is a diagram showing an example of the hardware configuration of the computer 1000.

As shown in Figure 10, the computer 1000 includes, for example, a processor 1001, a memory 1002, a storage device 1003, an input I/F unit 1004, a data I/F unit 1005, a communication I/F unit 1006, and a display device 1007.

The computer 1000 may be, for example, a server computer, a personal computer (for example, a desktop, a laptop, a tablet, or the like), a media computer platform (for example, a cable, a satellite set-top box, a digital video recorder, or the like), a handheld computer device (for example, a PDA, an e-mail client, or the like), another type of computer, or a communication platform.

The processor 1001 is a control unit that controls various processes in the computer 1000 by executing programs stored in the memory 1002.

The memory 1002 may be a storage medium such as a random access memory (RAM). The memory 1002 temporarily stores a program code of a program executed by the processor 1001 and data required when the program is executed.

The storage device 1003 is a non-volatile storage medium such as a hard disk drive (HDD) or a flash memory. The storage device 1003 stores an operating system and various programs for implementing each of the above configurations.

The input I/F unit 1004 is a device for receiving an input from a user. The input I/F unit 1004 is, for example, a keyboard, a mouse, a touch panel, various sensors, a wearable device, or the like. The input I/F unit 1004 may be connected to the computer 1000 via an interface such as a universal serial bus (USB).

The data I/F unit 1005 is a device for inputting data from the outside of the computer 1000. The data I/F unit 1005 is, for example, a drive device for reading data stored in various storage media. The data I/F unit 1005 may be provided outside the computer 1000. When the data I/F unit 1005 is provided outside the computer 1000, the data I/F unit 1005 is connected to the computer 1000 via an interface such as a USB.

The communication I/F unit 1006 is a device for performing data communication with an external device of the computer 1000 via a network such as the Internet, in a wired or wireless manner. The communication I/F unit 1006 may be provided outside the computer 1000. When the communication I/F unit 1006 is provided outside the computer 1000, the communication I/F unit 1006 is connected to the computer 1000 via an interface such as a USB.

The display device 1007 is a device for displaying various types of information. The display device 1007 is, for example, a liquid crystal display, an organic electro-luminescence (EL) display, a display for a wearable device, or the like. The display device 1007 may be provided outside the computer 1000. When the display device 1007 is provided outside the computer 1000, the display device 1007 is connected to the computer 1000 via, for example, a display cable. When a touch panel is adopted as the input I/F unit 1004, the display device 1007 may be configured integrally with the input I/F unit 1004.

One embodiment of the present invention has been described above. The sequence extraction system 100 can generate a plurality of candidate fusion sequences on the basis of each of a plurality of candidate insertion patterns, acquire structure prediction result information for each of a plurality of candidate fusion proteins corresponding to the plurality of candidate fusion sequences, evaluate an insertion site on the basis of a prediction result, and extract at least one candidate fusion sequence from the plurality of candidate fusion sequences on the basis of an evaluation result. Thereby, it is possible to easily extract an amino acid sequence of a protein suitable for three-dimensional structure analysis.

The sequence extraction system 100 can also evaluate whether an insertion site contributes to the formation of a secondary structure of a protein and extract at least one candidate fusion sequence in which it is evaluated that an insertion site contributes to the formation of a secondary structure of a protein. Thereby, it is possible to extract an amino acid sequence of a protein suitable for three-dimensional structure analysis, in which a target protein and an insertion polypeptide form a series of secondary structures near the insertion site.

The sequence extraction system 100 can also evaluate an insertion site on the basis of a score (PAE score) regarding an error of each of prediction results of each of a plurality of atoms included in a predicted structure indicated by structure prediction result information, relative to each of the prediction result of each of the plurality of atoms. Thereby, it is possible to easily extract an amino acid sequence of a protein suitable for three-dimensional structure analysis, with a limited variation in the orientation of a target protein and an insertion polypeptide.

In addition, the sequence extraction system 100 can further evaluate whether an insertion site contributes to the formation of a secondary structure of a protein on the basis of a PAE score. Thereby, it is possible to improve the accuracy of evaluation of whether an insertion site contributes to the formation of a secondary structure of a protein, and can extract an amino acid sequence of a protein suitable for three-dimensional structure analysis.

The sequence extraction system 100 can also evaluate whether an insertion site forms an α-helix structure.

The sequence extraction system 100 can also extract at least one candidate fusion sequence from a plurality of candidate fusion sequences in each of which a target protein is a membrane protein or a G protein-coupled receptor.

Note that this embodiment is intended to facilitate understanding of the present invention and is not intended to limit the interpretation of the present invention. The present invention may be modified/improved without departing from the spirit of the present invention, and equivalents thereof are also included in the present invention.

### Reference Signs List

- 100: Sequence extraction system
- 110: Storage unit
- 120: Sequence acquisition unit
- 130: Candidate sequence generation unit
- 140: Structure prediction result acquisition unit
- 150: Evaluation unit
- 160: Sequence extraction unit
- 170: Output unit
- 200: User terminal
- 300: Structure prediction system

## Claims

1. A sequence extraction system comprising:
a candidate sequence generation unit that generates a plurality of candidate fusion sequences, which are a plurality of candidates for a fusion sequence in which at least one inserted amino acid sequence corresponding to insertion polypeptide is inserted into an entirety or a part of a target amino acid sequence corresponding to a target protein, based on each of a plurality of candidate insertion patterns, which are a plurality of candidates for an insertion pattern for inserting at least one of the inserted amino acid sequence into the entirety or the part of the target amino acid sequence;
a structure prediction result acquisition unit that acquires structure prediction result information indicating a result of prediction of a structure of each of a plurality of candidate fusion proteins corresponding to the plurality of candidate fusion sequences respectively, based on each of the plurality of candidate fusion sequences;
an evaluation unit that evaluates an insertion site into which the inserted amino acid sequence is inserted, based on the result of the prediction; and
a sequence extraction unit that extracts at least one candidate fusion sequence from the plurality of candidate fusion sequences, based on the result of the evaluation.

2. The sequence extraction system according to claim 1, wherein
the evaluation unit evaluates whether the insertion site contributes to the formation of a secondary structure of a protein in the candidate fusion protein, and
the sequence extraction unit extracts the at least one candidate fusion sequence of which the structure prediction result acquisition unit evaluates that the insertion site contributes to the formation of the secondary structure.

3. The sequence extraction system according to claim 2, wherein the evaluation unit evaluates the insertion site, based on a score regarding an error of each prediction result of each of a plurality of atoms included in a predicted structure indicated by the structure prediction result information, relative to each of the prediction result of each of the plurality of atoms.

4. The sequence extraction system according to claim 3, wherein the evaluation unit evaluates whether the insertion site contributes to the formation of the secondary structure, further based on the score.

5. The sequence extraction system according to claim 1, wherein the evaluation unit evaluates the insertion site, based on an angle determined by orientation of a first helix and a second helix of the target protein which are connected to the insertion polypeptide.

6. The sequence extraction system according to claim 1, wherein the evaluation unit evaluates the insertion site, based on a distance between the insertion site and a lipid bilayer predicted when the target protein is embedded in the lipid bilayer.

7. The sequence extraction system according to any one of claims 2 to 6, wherein the evaluation unit evaluates whether the insertion site contributes to the formation of a secondary structure in the candidate fusion protein in accordance with a hydrogen bond pattern predicted based on coordinates of atoms in the predicted structure.

8. The sequence extraction system according to any one of claims 2 to 4, wherein the secondary structure is an α helix structure.

9. The sequence extraction system according to any one of claims 1 to 6, wherein the target protein is a membrane protein.

10. The sequence extraction system according to any one of claims 1 to 6, wherein the target protein is a G protein-coupled receptor.

11. A sequence extraction method executed by a computer, the method comprising:
generating a plurality of candidate fusion sequences, which are a plurality of candidates for a fusion sequence in which at least one inserted amino acid sequence corresponding to insertion polypeptide is inserted into an entirety or a part of a target amino acid sequence corresponding to a target protein, based on each of a plurality of candidate insertion patterns, which are a plurality of candidates for an insertion pattern for inserting at least one of the inserted amino acid sequence into the entirety or the part of the target amino acid sequence;
acquiring structure prediction result information indicating a result of prediction of a structure of each of a plurality of candidate fusion proteins corresponding to the plurality of candidate fusion sequences respectively, based on each of the plurality of candidate fusion sequences;
evaluating an insertion site into which the inserted amino acid sequence is inserted, based on the result of the prediction; and
extracting at least one candidate fusion sequence from the plurality of candidate fusion sequences, based on the result of the evaluation.

12. A sequence extraction program causing a computer to implement:
a candidate sequence generation unit that generates a plurality of candidate fusion sequences, which are a plurality of candidates for a fusion sequence in which at least one inserted amino acid sequence corresponding to insertion polypeptide is inserted into an entirety or a part of a target amino acid sequence corresponding to a target protein, based on each of a plurality of candidate insertion patterns, which are a plurality of candidates for an insertion pattern for inserting at least one of the inserted amino acid sequence into the entirety or the part of the target amino acid sequence;
a structure prediction result acquisition unit that acquires structure prediction result information indicating a result of prediction of a structure of each of a plurality of candidate fusion proteins corresponding to the plurality of candidate fusion sequences respectively, based on each of the plurality of candidate fusion sequences;
an evaluation unit that evaluates an insertion site into which the inserted amino acid sequence is inserted, based on the result of the prediction; and
a sequence extraction unit that extracts at least one candidate fusion sequence from the plurality of candidate fusion sequences, based on the result of the evaluation.
